Europäisches Patentamt

(19) European Patent Office

Office européen des brevets

(11) Publication number: **0 279 542 B1**

# (12) EUROPEAN PATENT SPECIFICATION

(45) Date of publication of patent specification:
**21.08.91 Bulletin 91/34**

(51) Int. Cl.[5]: **C07C 229/24, C07C 227/38**

(21) Application number: **88300742.9**

(22) Date of filing: **28.01.88**

(54) **Novel aspartic acid crystals and a process for the production thereof.**

(30) Priority: **19.02.87 JP 36604/87**

(43) Date of publication of application:
**24.08.88 Bulletin 88/34**

(45) Publication of the grant of the patent:
**21.08.91 Bulletin 91/34**

(84) Designated Contracting States:
**CH DE FR GB IT LI NL**

(56) References cited:
**DE-A- 2 613 564**
**US-A- 3 183 263**

(73) Proprietor: **AJINOMOTO CO., INC.**
**5-8, Kyobashi 1-chome, Chuo-ku**
**Tokyo 104 (JP)**

(72) Inventor: **Yukawa, Toshihide**
**No. 1-1, Suzuki-cho**
**Kawasaki-ku Kawasaki-shi Kanagawa-ken**
**(JP)**
Inventor: **Nakamura, Masao**
**No. 1-1, Suzuki-cho**
**Kawasaki-ku Kawasaki-shi Kanagawa-ken**
**(JP)**
Inventor: **Takeda, Hideo**
**No. 1-1, Suzuki-cho**
**Kawasaki-ku Kawasaki-shi Kawasaki-ken (JP)**
Inventor: **Takahashi, Satoji**
**No.1730, Ohazahinaga**
**Yokkaichi-shi (JP)**

(74) Representative: **Bond, Bentley George et al**
**HASELTINE LAKE & CO. Hazlitt House 28**
**Southampton Buildings Chancery Lane**
**London, WC2A 1AT (GB)**

## Description

The present invention relates to a novel crystal form of L-aspartic acid (hereinbefore referred to as L-Asp) and a process for the production thereof. L-Asp is useful as a starting material for the production of the sweetening agent Aspartame.

There is known a process for the production of L-Asp which generally comprises neutralization and crystallization of a solution obtained by an enzymatic reaction of fumaric acid.

However, L-Asp obtained by this process consists of α-type crystals having a scale or flake form. Therefore, the crystals separated by a centrifugal separator contain adsorbed water in an amount as high as 15-18% and also inevitably incorporate large amounts of impurities from the crystallization medium due to this absorbed water, and thus the purity of the obtained L-Asp is deteriorated.

Accordingly, the present inventors have intensively studied various crystallization conditions and, as a result, have come to discover a process for obtaining a novel β-type crystals of L-Asp in a cylindrical form by adding a small amount of L-phenylalanine (hereinafter referred to as L-Phe) in the presence of sodium chloride.

According to the present invention, there is provided β-type crystals of aspartic acid which exhibit X-ray diffraction peaks at angles of diffraction (2θ, Cu kα line) of at least 18.8°, 19.7° and 25.0° when measured by a powder X-ray diffraction method.

The present invention also provides a process for the production of β-type crystals of aspartic acid, which comprises crystallizing L-Asp in the co-presence of sodium chloride and phenylalanine from an aqueous solution.

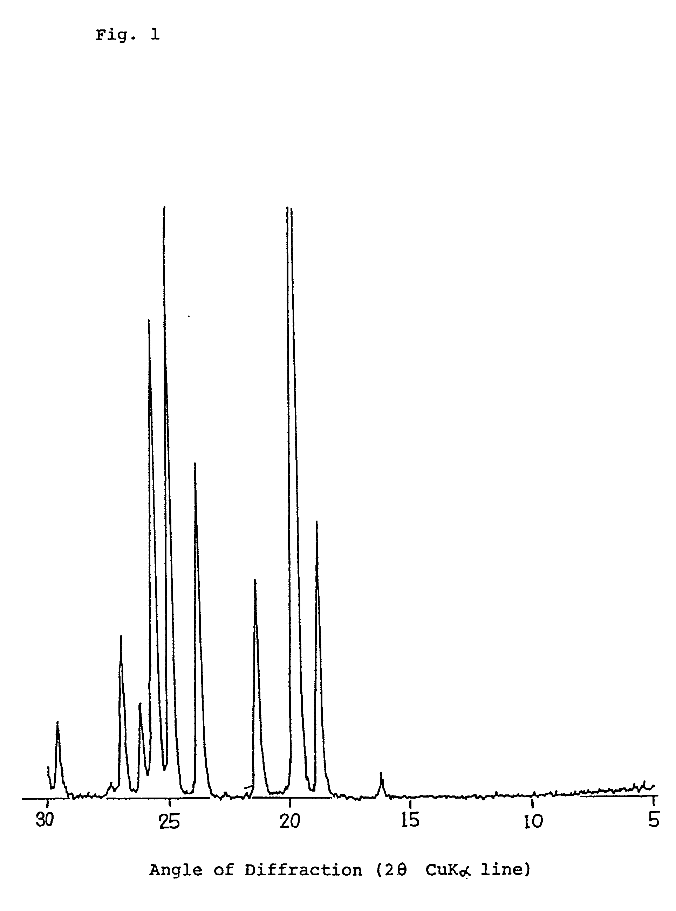

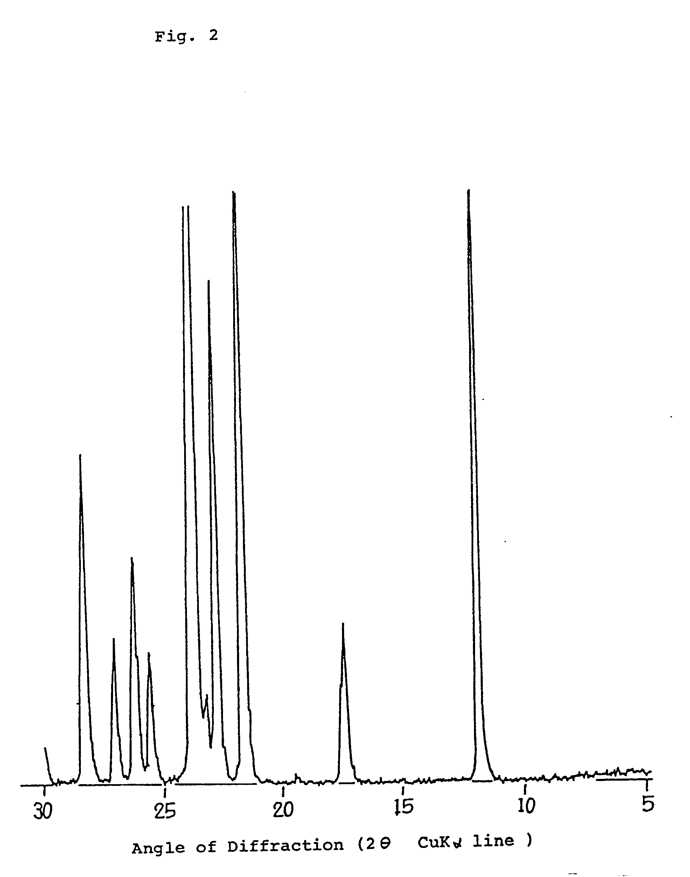

Reference will be made hereinbelow to the drawings in which figures 1 and 2 are X-ray diffraction figures as measured by a powder diffraction X-ray method, Figure 1 being for β-type crystals and Figure 2 being for α-type crystals.

The β-type crystals contain adsorbed water in an amount of as low as 4-5% when separated by a centrifugal separator, and as a result, high purity L-Asp may be obtained with only a limited extent of incorporation of impurities. Further, drying of the obtained hydrous L-Asp is easier with the β-type crystals than with α-type crystals because the amount of adsorbed water is lower in the former case and thus it is economically more advantageous.

One method for obtaining the β-type crystals comprises adding to a solution of L-Asp sodium chloride at a concentration of from 5% by weight to the saturation point, preferably 15-25% by weight, dissolving the sodium chloride, then adding and dissolving L-Phe and thereafter crystallizing L-Asp by cooling or neutralization.

Although the amount of L-Phe added is not particularly restricted, it can be seen, as demonstrated in Example 1, that even an amount of as small as 0.02% by weight can exert an effect. In general, the range of 0.01-0.1% by weight is suitable. The obtained β-type crystals exhibit diffraction angles (2θ, CuKα line) of at least 18.8°, 19.7° and 25.0° when measured by a powder diffraction X-ray method.

According to the present invention, as described above, by adding a small amount of L-Phe in the presence of sodium chloride during crystallization of L-Asp, industrially advantageous high purity L-Asp may be obtained.

The solutions of L-Asp may be any of those from which L-Asp has been previously obtained and crystallized, preferably an aqueous solution. The pH of the solution may be constant or variably adjusted and may preferably be from 2-8, more preferably 3-5.5.

The invention now being generally described, the same will be better understood by reference to certain specific examples which are included herein for purposes of illustration only and are not intended to be limiting of the invention or any embodiment thereof, unless specified.

### Example 1

One liter of water was added to 150 g of L-Asp and 200 g of sodium chloride, and caustic soda was added thereto with stirring to adjust to pH 5.5 whereby L-Asp was dissolved. Then, 270 mg of L-Phe was added and dissolved, and thereafter 35% hydrochloric acid was added to adjust to pH 3, whereby L-Asp was crystallized by neutralization. The resultant crystals were separated by a centrifugal separator. The obtained crystals were cylindrical crystals and the adsorbed water thereof was 4.5%. Thereafter, the hydrous crystals were dried, and the powder X-ray pattern was measured to exhibit a structure of β-type crystals (see FIGURE 1).

### Example 2

One liter of water was added to 100 g of L-Asp and 250 g of sodium chloride, and 35% hydrochloric acid was added thereto with stirring to dissolve L-Asp.

Thereafter, 540 mg of L-Phe was added and dissolved, and thereafter caustic soda was added to adjust the pH to 3, whereby L-Asp was crystallized by neutralization. The resultant crystals were cylindrical crystals and the adsorbed water thereof was 4.0%. Thereafter, the hydrous crystals were dried and the powder X-ray pattern was measured to exhibit a structure of β-type crystals.

### Comparative Example 1

One liter of water was added to 150 g of L-Asp and 200 g of sodium chloride, and caustic soda was added thereto with stirring to adjust to pH 5.5 whereby L-Asp was dissolved. After confirming that L-Asp had been dissolved, 35% hydrochloric acid was added to adjust the pH to 3, whereby L-Asp was crystallized by neutralization. The resultant crystals were separated by a centrifugal separator. The obtained crystals were flake-formed crystals and the water adsorbed thereto was 16.5%. Thereafter, the hydrous crystals were dried, and the powder X-ray pattern was measured to exhibit a structure of α-type crystals.

The invention now being fully described, it will be apparent to one of ordinary skill in the art that many changes and modifications can be made thereto without departing from the spirit or scope of the invention as set forth herein.

## Claims

1. β-type crystals of aspartic acid which exhibit X-ray diffraction peaks at angles of diffraction (2θ, Cu kα line) of at least 18.8°, 19.7° and 25.0° when measured by a powder X-ray diffraction method.

2. Crystals as claimed in claim 1, wherein the amount of adsorbed water in said crystals is at most 5% by weight.

3. A process for the production of β-type crystals of aspartic acid, which comprises crystallizing L-Asp in the co-presence of sodium chloride and phenylalanine from an aqueous solution.

4. A process according to claim 3, wherein the amount of sodium chloride ranges from a concentration of 5% by weight to the saturation point of sodium chloride in said aqueous solution.

5. A process according to claim 4, wherein the concentration of sodium chloride ranges from 15 to 25% by weight.

6. A process according to any of claims 3 to 5, wherein the amount of L-phenylalanine which is present in said aqueous solution ranges from 0.01 to 0.1% by weight.

7. A process according to any of claims 3 to 6, which further comprises separating said crystallized L-Asp from said aqueous solution.

## Patentansprüche

1. Asparaginsäurekristalle vom β-Typ, die Röntgenbeugungsspitzen bei Beugungswinkeln (2θ, Cu-kα-Linie) von mindestens 18,8°, 19,7° und 25,0° bei Messung durch ein Röntgenbeugungspulverfahren aufweisen.

2. Kristalle nach Anspruch 1, wobei die Menge an adsorbïertem Wasser in den Kristallen höchstens 5 Gew.-% beträgt.

3. Verfahren zur Herstellung von Asparaginsäurekristallen vom β-Typ, bei dem man L-Asp bei gleichzeitiger Anwesenheit von Natriumchlorid und Phenylalanin aus einer wäßrigen Lösung auskristallisiert.

4. Verfahren nach Anspruch 3, wobei die Menge an Natriumchlorid im Bereich zwischen einer Konzentration von 5 Gew.-% bis zum Sättigungspunkt an Natriumchlorid in der wäßrigen Lösung liegt.

5. Verfahren nach Anspruch 4, wobei die Konzentration an Natriumchlorid im Bereich von 15 bis 25 Gew.-% liegt.

6. Verfahren nach einem der Ansprüche 3 bis 5, wobei die Menge an L-Phenylalanin, das in der wäßrigen Lösung anwesend ist, im Bereich von 0,01 bis 0,1 Gew.-% liegt.

7. Verfahren nach einem der Ansprüche 3 bis 6, bei dem man weiterhin die auskristallisierte L-Asp aus der wäßrigen Lösung abtrennt.

## Revendications

1. Cristaux d'acide aspartique de type β qui présentent des pics de diffraction des rayons X à des angles de diffraction (2θ, raie CuKα) d'au moins 18,8°, 19,7° et 25,0° lorsqu'on les mesure selon la méthode de diffraction des rayons X par les poudres.

2. Cristaux selon la revendication 1, dans lesquels la quantité d'eau adsorbée dans lesdits cristaux est d'au plus 5% en poids.

3. Procédé pour la production de cristaux d'acide aspartique de type β, qui comprend la cristallisation du L-Asp en coprésence de chlorure de sodium et de phénylalanine à partir d'une solution aqueuse.

4. Procédé selon la revendication 3, dans lequel la quantité de chlorure de sodium se situe entre une concentration de 5% en poids et le point de saturation du chlorure de sodium dans ladite solution aqueuse.

5. Procédé selon la revendication 4, dans lequel la concentration du chlorure de sodium se situe dans la gamme de 15 à 25% en poids.

6. Procédé selon l'une quelconque des revendications 3 à 5, dans lequel la quantité de L-phénylalanine qui est présente dans ladite solution aqueuse se situe dans la gamme de 0,01 à 0,1% en poids.

7. Procédé selon l'une quelconque des revendications 3 à 6, qui comprend de plus la séparation dudit L-Asp cristallisé d'avec ladite solution aqueuse.

Fig. 1

Angle of Diffraction (2θ CuKα line)

Fig. 2

Angle of Diffraction (2θ  CuKα line )